# EUROPEAN PATENT APPLICATION

(11) **EP 2 455 103 A1**
(43) Date of publication of application: **23.05.2012**
(21) Application number: 10799859.3
(22) Date of filing: 14.07.2010
(51) Int. Cl.: A61K 45/00, A61K 31/7088, A61P 35/00

(54) **ANTI-CANCER AGENT, METHOD FOR INDUCING APOPTOSIS OF CANCER CELLS, AND METHOD FOR SCREENING FOR ANTI-CANCER AGENT**

(30) Priority: 15.07.2009 JP 2009166663
(71) Applicant: Medical Care Proteomics Biotechnology Co., Ltd., Ishikawa 920-0293 (JP)
(72) Inventor: ISHIGAKI, Yasuhito, Kahoku-gun Ishikawa 920-0293 (JP); TOMOSUGI, Naohisa, Kahoku-gun Ishikawa 920-0293 (JP)
(74) Representative: KATZAROV S.A.
(86) International application number: PCT/JP2010/061895
(87) International publication number: WO 2011/007801

(57) **Abstract**

Disclosed is an anti-cancer agent which can inhibit the progression of cell cycle in the M phase, unlike taxane anti-cancer agents. The anti-cancer agent comprises a compound capable of inhibiting specifically the expression and/or functions of RBM8A depicted in SEQ ID NO: 1, Magoh depicted in SEQ ID NO: 3 or an isoform thereof. The anti-cancer agent can inhibit the expression and/or functions of RBM8A or Magoh in cancer cells to terminate the cell cycle in the M phase, thereby inhibiting the proliferation of the cells. Therefore, the anti-cancer agent can inhibit the proliferation of cancer cells and induce the apoptosis of cancer cells, leading to the cure of cancer.

## Description

### TECHNICAL FIELD

The present invention relates to an anti-cancer agent capable of specifically inducing the cell death of cancer cells by inhibiting the expression of RBM8A or Magoh that is an intranuclear protein, a method for inducing apoptosis of cancer cells using this anti-cancer agent, a pharmaceutical composition containing the anti-cancer agent as an active ingredient, and a method for screening for an anti-cancer agent by measuring the amount of expression of RBM8A or Magoh.

### BACKGROUND ART

In the field of diagnosis and therapy of cancer, proteins that are specifically expressed in cells of the cancer have been identified so far by a variety of proteomics analyses. Conventionally, investigations of cancer treatments targeting these proteins have focused on enhancement or repression of the expression and/or activity of the protein.

Human RBM8A (RNA binding motif protein 8A) that is an intranuclear protein set out in SEQ ID NO: 1 is a comparatively low molecular weight protein, and reportedly has a homology with Drosophila melanogaster Y14 on which interactions with various factors have been reported. In this regard, Y14 is reported to have an RNA binding motif, and binds to an RNA after forming a heterodimer with Magoh that is another intranuclear protein. Cell proliferation in Drosophila melanogaster culture cells is reportedly inhibited by way of inhibition of the expression of Y14. However, regarding proteins homologous to Y14 in other organism species, their functions have been unknown, and in particular, any report regarding functions and relationship in connection with DNA replication and cell division have not been made. Accordingly, it is not evident as to which phase of the cell cycle is inhibited during the cell proliferation (see Nonpatent Document 1). Particularly, solely causing suppression of cell proliferation is not sufficient for use as an anti-cancer agent, and induction of cell death such as apoptosis is essential for use as an anti-cancer agent. Therefore, it is not elucidated as to whether or not a factor that inhibits a homologous protein to Y14 in other organisms can be utilized in a pharmaceutical composition which includes an anti-cancer agent or the like.

On the other hand, human Magoh that is a protein set out in SEQ ID NO: 5 is known to bind to an RNA after forming a heterodimer with human RBM8A. However, it is not elucidated as to whether or not a factor that inhibits Magoh can be utilized in a pharmaceutical composition which includes an anti-cancer agent or the like.

At present, RNA interference (RNAi) techniques are frequently used in investigations in life science field, and the usability thereof has been broadly recognized. In this regard, RNAi refers to a phenomenon of degradation of an mRNA by a double strand RNA in a manner specific for the sequence thereof, consequently leading to suppression of the gene expression. Since 2001 when siRNA (small interference RNA) that is a low molecular double strand RNA having 21 bases was reported to be capable of mediating RNAi in mammalian animal cells, siRNA has been frequently used as a method for suppressing expression of a target gene. Furthermore, in recent years, selection criteria of siRNA sequence for achieving higher effects in RNAi were also reported (see Nonpatent Documents 2 and 3).

RNAi techniques are expected for application to medical drugs and treatments of various intractable diseases including cancer. For example, Patent Document 1 discloses a therapeutic agent for interstitial lung disease characterized by including as an active ingredient a substance that suppresses expression of a certain protein, such as siRNA.

Conventionally, many of anti-cancer agents have an activity of specifically inhibiting progression of cell cycles in the S phase of the cell cycles. To the contrary, taxane anti-cancer agents such as paclitaxel have been known to inhibit the progression of cell cycles in the M phase by binding to microtubules to inhibit depolymerization of the microtubules. Accordingly, anti-cancer agents that inhibit the progression of cell cycles in the M phase are expected to be broadly usable for cancer therapy.

Patent Document 1: Japanese Unexamined Patent Application, Publication No. 2007-319009

Nonpatent Document 1: Herve Le Hir et al., "The protein Mago provides a link between splicing and mRNA", EMBO reports, 12, p. 1119-1124 (2001)

Nonpatent Document 2: Elbashir S. M. et al., "Duplexes of 21-nucleotide RNAs mediate RNA interference in cultured mammalian cells", Nature, 411(6836), p. 494-498 (2001)

Nonpatent Document 3: Reynolds A. et al., "Rational siRNA design for RNA interference", Nature Biotechnol., 22(3), p. 326-330 (2004)

### DISCLOSURE OF THE INVENTION

### Problems to be Solved by the Invention

However, taxane anti-cancer agents such as paclitaxel are concerned about having side effects such as decrease of leukocytes and neutrophils, injury of peripheral nerves, nausea, vomiting and the like. Therefore, development of an anti-cancer agent that inhibits progression of cell cycles in the M phase other than the taxane anti-cancer agents has been desired.

### Means for Solving the Problems

The present inventors thoroughly investigated in view of the foregoing problems. Consequently, it was found that RBM8A that is a protein homologous to Y14 in human is localized in centrosome, spindle fiber and contractile ring, and inhibition of RBM8A expression leads to inhibition of normal polymerization of tubulin to allow cancer cells in the M phase to be enriched, and that inhibition of RBM8A expression enables apoptosis to be caused in cells enriched in the M phase. In addition, it was also found that inhibition of Magoh expression results in suppression of RBM8A expression, whereby apoptosis is caused. Based on such findings, the present inventors revealed that a substance that inhibits RBM8A or Magoh expression can be utilized as an anti-cancer agent, and thus the present invention was completed.

Specifically, the present invention provides the following.

A first aspect of the invention provides an anti-cancer agent including a compound that specifically suppresses expression and/or a function of a protein set out in SEQ ID NO: 1 or an isoform thereof.

The anti-cancer agent according to the first aspect is capable of specifically inhibiting expression and/or a function of RBM8A set out in SEQ ID NO: 1 or an isoform thereof. In this regard, since inhibition of expression and/or a function of RBM8A in cancer cells allows the cell cycles to be arrested at the M phase, thereby capable of inhibiting growth of the cells, proliferation of cancer cells can be suppressed, and concurrently apoptosis of the cancer cells can be induced, leading to cure of the cancer.

As referred to herein, the term to "specifically suppress" means to suppress only expression and/or a function of a protein set out in SEQ ID NO: 1 in a living body by an anti-cancer agent, without exhibiting an off-target effect.

A second aspect of the invention provides the anti-cancer agent according to the first aspect, in which the compound is a double strand siRNA that specifically suppresses the expression of the protein set out in SEQ ID NO: 1 or an isoform thereof, and at least one of RNA molecules that constitute the double strand siRNA is capable of complementarily binding to an mRNA set out in SEQ ID NO: 2.

According to the invention provided by the second aspect, the compound that specifically suppresses the expression of the protein set out in SEQ ID NO: 1 or an isoform thereof is defined as a double strand siRNA. A double strand siRNA can suppress the expression of the protein set out in SEQ ID NO: 1 or an isoform thereof as long as it has an RNA strand that is capable of complementarily binding to an mRNA set out in SEQ ID NO: 2 encoding a protein set out in SEQ ID NO: 1; therefore, it can be suitably used as the anti-cancer agent according to the first aspect.

As referred to herein, "siRNA" means a short strand RNA molecule capable of mediating RNAi, and having 21 to 27 bases.

A third aspect of the invention provides an anti-cancer agent including a compound that specifically suppresses expression and/or a function of a protein set out in SEQ ID NO: 3 or an isoform thereof.

The anti-cancer agent according to the third aspect is capable of specifically inhibiting expression and/or a function of Magoh set out in SEQ ID NO: 3 or an isoform thereof. In this regard, inhibition of expression and/or a function of Magoh in cancer cells allows the expression and/or a function of RBM8A to be also inhibited. Therefore, proliferation of cancer cells can be suppressed, and concurrently apoptosis of the cancer cells can be induced, leading to cure of the cancer.

A fourth aspect of the invention provides the anti-cancer agent according to the third aspect, in which the compound is a double strand siRNA that specifically suppresses the expression of the protein set out in SEQ ID NO: 3 or an isoform thereof, and at least one of RNA molecules that constitute the double strand siRNA is capable of complementarily binding to an mRNA set out in SEQ ID NO: 4.

According to the invention provided by the fourth aspect, the compound that specifically suppresses the expression of the protein set out in SEQ ID NO: 3 or an isoform thereof is defined as a double strand siRNA. A double strand siRNA can suppress the expression of the protein set out in SEQ ID NO: 3 or an isoform thereof as long as it has an RNA strand that is capable of complementarily binding to an mRNA set out in SEQ ID NO: 4 encoding a protein set out in SEQ ID NO: 3; therefore, it can be suitably used as the anti-cancer agent according to the third aspect.

A fifth aspect of the invention provides the anti-cancer agent according to any one of the first to fourth aspects, for use in a treatment of non-small cell lung cancer or breast cancer.

The anti-cancer agent of the present invention may be suitably used in a treatment of particularly non-small cell lung cancer and breast cancer. Therefore, according to the invention provided by the fifth aspect, proliferation of cancer cells is specifically suppressed, whereby apoptosis of cancer cells can be specifically induced.

A sixth aspect of the invention provides a pharmaceutical composition containing as an active ingredient the anti-cancer agent according to any one of the first to fifth aspects.

In the invention provided by the sixth aspect, the invention according to the first to fifth aspects is defined in terms of an invention directed to a pharmaceutical composition. Therefore, according to the invention provided by the sixth aspect, effects similar to those of the invention according to the first to fifth aspects can be achieved.

A seventh aspect of the invention provides a method for inducing apoptosis of cancer cells, the method including using the anti-cancer agent according to any one of the first to fifth aspects.

In the invention provided by the seventh aspect, the invention according to the first to fifth aspects is defined in terms of an invention directed to a method for inducing apoptosis of cancer cells. Therefore, according to the invention provided by the seventh aspect, effects similar to those of the invention according to the first to fifth aspects can be achieved.

An eighth aspect of the invention provides a method for screening for an anti-cancer agent, the method including: allowing isolated cancer cells and candidate substances to be in contact; assaying expression and/or a function of a protein set out in SEQ ID NO: 1 or 3 or an isoform thereof in the cancer cells; and selecting a candidate substance that reduces the expression and/or the function of the protein set out in SEQ ID NO: 1 or 3 or an isoform thereof from the candidate substances.

A ninth aspect of the invention provides the method for screening for an anti-cancer agent according to the eighth aspect, in which: interaction or complex formation of the protein set out in SEQ ID NO: 1 or 3, and other protein that interacts with the protein set out in SEQ ID NO: 1 or 3 in a living body is detected in the step of assaying expression and/or a function of a protein set out in SEQ ID NO: 1 or 3 or an isoform thereof; and a candidate substance that reduces the interaction or complex formation of the protein set out in SEQ ID NO: 1 or 3 and the other protein is selected in the step of selecting a candidate substance that reduces the expression and/or the function of the protein set out in SEQ ID NO: 1 or 3 or an isoform thereof from the candidate substances.

A tenth aspect of the invention provides the method for screening for an anti-cancer agent according to the eighth aspect, in which: an expression level of a gene that expresses an mRNA set out in SEQ ID NO: 2 or 4 is measured in the step of assaying expression and/or a function of a protein set out in SEQ ID NO: 1 or 3 or an isoform thereof; and a candidate substance that decreases an expression level of a gene that expresses an mRNA set out in SEQ ID NO: 2 or 4 is selected in the step of selecting a candidate substance that reduces the expression and/or the function of the protein set out in SEQ ID NO: 1 or 3 or an isoform thereof from the candidate substances.

According to the invention provided by the eighth to tenth aspects, a method for screening for an anti-cancer agent is defined with an inhibitory activity of expression and/or a function of RBM8A set out in SEQ ID NO: 1, or Magoh set out in SEQ ID NO: 3 as a marker. As described above, since inhibition of RBM8A expression in cancer cells allows the cell cycles to be arrested at the M phase, thereby capable of suppressing proliferation of cells, and apoptosis can be induced. In addition, inhibition of Magoh expression in cancer cells enables the proliferation of cells to be suppressed, and apoptosis can be induced. Therefore, according to the invention provided by the eighth to tenth aspects, screening of an anti-cancer agent capable of inhibiting proliferation of cells is enabled via inhibition of RBM8A or Magoh expression.

An eleventh aspect of the invention provides the method for screening for an anti-cancer agent according to the twelfth aspect, in which the candidate substance is a double strand siRNA having a 3' overhang, and at least one of RNA molecules that constitute the double strand siRNA is capable of complementarily binding to mRNA set out in SEQ ID NO: 2 or 4.

The invention provided by the eleventh aspect is a method for screening for an anti-cancer agent, in which a double strand siRNA having an RNA molecule capable of complementarily binding to an mRNA encoding RBM8A or Magoh is the candidate substance. Since a double strand siRNA having an RNA molecule capable of complementarily binding to an mRNA encoding a protein tends to inhibit the expression of the protein, efficient screening of an anti-cancer agent is enabled by using the double strand siRNA as the candidate substance.

A twelfth aspect of the invention provides the method for screening for an anti-cancer agent according to any one of the eighth to eleventh aspects, in which the cancer cells are cultured in the presence of the candidate substance in the step of allowing isolated cancer cells and candidate substances to be in contact.

A thirteenth aspect of the invention provides the method for screening for an anti-cancer agent according to any one of the eighth to twelfth aspects, in which expression and/or a function of the protein is determined using an antibody to the protein set out in SEQ ID NO: 1 or 3 in the step of assaying expression and/or a function of a protein set out in SEQ ID NO: 1 or 3 or an isoform thereof.

According to the invention provided by the twelfth and the thirteenth aspects, specific procedures of the invention according to the eighth to eleventh aspects are defined. Therefore, according to the invention provided by the twelfth and the thirteenth aspects, effects similar to those of the invention according to the eighth to eleventh aspects can be achieved.

### Effects of the Invention

The anti-cancer agent of the present invention can specifically inhibit expression and/or a function of RBM8A set out in SEQ ID NO: 1, Magoh set out in SEQ ID NO: 5, or an isoform thereof. Inhibition of expression and/or a function of RBM8A in cancer cells allows the cell cycles to be arrested at the M phase, thereby capable of inhibiting proliferation of cells. In addition, inhibition of Magoh expression in cancer cells enables the proliferation of cells to be inhibited. Therefore, proliferation of cancer cells can be suppressed, and concurrently apoptosis of the cancer cells can be induced, leading to cure of the cancer.

### BRIEF DESCRIPTION OF THE DRAWINGS

Fig. 1 shows views illustrating intracellular localization of RBM8A.
Fig. 2 shows views illustrating intracellular localization of RBM8A.
Fig. 3 shows optical microscopic views illustrating cells transfected with RBM8A-siRNA (IG-B).
Fig. 4 shows views illustrating results of immunostaining of cells transfected with each RBM8A-siRNA (1) to (3), using an anti-phosphorylated histone H3 antibody.
Fig. 5 shows a view illustrating the proportion of cells stained with the anti-phosphorylated histone H3 antibody (cells in M phase) with respect to cells stained with DAPI.
Fig. 6 shows a view illustrating the expression level of RBM8A in cells transfected with each RBM8A-siRNA (1) to (3).
Fig. 7 shows views illustrating results of flow cytometry of cells transfected with RBM8A-siRNA (2).
Fig. 8 shows phase contrast microscopic views illustrating cells transfected with RBM8A-siRNA (2).
Fig. 9 shows a view illustrating results of immunostaining of cells transfected with RBM8A-siRNA (2), using an anti-γ-tubulin antibody.
Fig. 10 shows a view illustrating the proportion of cells having abnormal microtubular shape in cells transfected with each RBM8A-siRNA (1) to (3).
Fig. 11 shows a view illustrating the activity of caspase 3/7 in cells transfected with each RBM8A-siRNA (1) to (3).
Fig. 12 shows a view illustrating the expression level of RBM8A in cells transfected with each RBM8A-siRNA (IG-B) or (IG-F).
Fig. 13 shows a view illustrating the Giemsa stained image of cells transfected with each RBM8A-siRNA (IG-B) or (IG-F).
Fig. 14 shows a view illustrating the expression level of RBM8A and Magoh in cells transfected with Magoh-siRNA (1) or (2) .
Fig. 15 shows views illustrating results of analysis of the cell cycle of cells transfected with Magoh-siRNA (1) or (2) .

### PREFERRED MODE FOR CARRYING OUT THE INVENTION

Hereinafter, embodiments of the present invention are explained in detail.

### Anti-cancer Agent

The anti-cancer agent of the present invention includes a compound that specifically suppresses the expression and/or function of a protein set out in SEQ ID NO: 1 or 3.

Typical compound described above may include, for example, a double strand siRNA that specifically suppress the expression of a protein set out in SEQ ID NO: 1 or 3, in which at least one of RNA molecules that constitute the double strand siRNA is capable of complementarily binding to a mRNA set out in SEQ ID NO: 2 or 4.

As described later, RBM8A that is a protein set out in SEQ ID NO: 1 is localized in cell nuclei, centrosome, spindle fiber, and contractile ring. In cancer cells, inhibition of RBM8A expression results in cell cycle arrest at the M phase, thereby leading to cell death of the cancer cells due to apoptosis. Therefore, by specifically inhibiting the expression and/or function of RBM8A that is the protein set out in SEQ ID NO: 1, proliferation of cancer cells can be suppressed, and concurrently apoptosis of the cancer cells can be induced.

Whereas, Magoh that is a protein set out in SEQ ID NO: 3 is capable of forming a heterodimer with RBM8A. Hence, inhibition of the Magoh expression in cancer cells results in cell death of the cancer cells due to apoptosis. Therefore, the expression and/or function of Magoh that is the protein set out in SEQ ID NO: 3 can be specifically inhibited, whereby proliferation of cancer cells can be suppressed, and concurrently apoptosis of the cancer cells can be induced.

Exemplary anti-cancer agent of the present invention that inhibits RBM8A may include a double strand siRNA obtained by pairing an RNA strand that includes an RNA set out in any one of SEQ ID NOs: 5 to 9 having an overhang consisting of a thymidine ribonucleotide dimer at the 3' end, with an RNA strand that includes a complementary strand of the RNA set out in any one of SEQ ID NOs: 5 to 9 having an overhang consisting of a thymidine ribonucleotide dimer at the 3' end.

The double strand siRNA can specifically bind to the mRNA set out in SEQ ID NO: 2 encoding RBM8A; therefore, the anti-cancer agent including this double strand siRNA can specifically suppress RBM8A expression that is a protein set out in SEQ ID NO: 1 in cells of cancer.

Also, exemplary anti-cancer agent of the present invention that inhibits Magoh may include a double strand siRNA obtained by pairing an RNA strand that includes an RNA set out in SEQ ID NO: 10 or 11 having an overhang consisting of a thymidine ribonucleotide dimer at the 3' end, with an RNA strand that includes a complementary strand of the RNA set out in SEQ ID NO: 10 or 11 having an overhang consisting of a thymidine ribonucleotide dimer at the 3' end.

The double strand siRNA can specifically bind to the mRNA set out in SEQ ID NO: 4 encoding Magoh; therefore, the anti-cancer agent including this double strand siRNA can specifically suppress Magoh expression that is a protein set out in SEQ ID NO: 3 in cells of cancer.

Herein, the double strand siRNA that constitutes the anti-cancer agent of the present invention may be produced chemically or enzymatically in vitro, or may be synthesized in vivo. Although the production method is not particularly limited, production by chemical synthesis according to a conventionally well-known method is preferred. In the case of a chemically synthesized double strand siRNA, operations for adjusting the concentration of the double strand siRNA, and for preventing contamination with saprophyte can be facilitated, and a double strand siRNA that exhibits high safety and a superior RNA interference effect can be obtain. For example, in the case of a double strand siRNA in which one complementary strand of 19 base pairs is set out in SEQ ID NO: 5, and a 3' overhang of 2 bases is a dimmer of thymidine ribonucleotides, chemical synthesis of: a sequence of an RNA strand set out in SEQ ID NO: 5 to which thymidine ribonucleotides of 2 bases were added at 3' end; and a sequence of an RNA strand that complementarily binds to the RNA strand set out in SEQ ID NO: 5, to which thymidine ribonucleotides of 2 bases were added at 3' end is each carried out, and these two RNA strands are submitted to pairing under conditions that enable the pairing.

Diseases to which the anti-cancer agent of the present invention is applied are not particularly limited as long as it is cancer, and non-small cell lung cancer, breast cancer, ovary cancer, stomach cancer, and esophageal cancer may be included. Of these, the anti-cancer agent is preferably applied to non-small cell lung cancer and breast cancer.

### Pharmaceutical Composition

The present invention also relates to a pharmaceutical composition containing the anti-cancer agent of the present invention as an active ingredient. The anti-cancer agent of the present invention may be used for cells and tissues in vitro, and it can be clinically applied also to human. When clinically applied to a human, a pharmaceutically acceptable carrier is preferably mixed if necessary to prepare a form of a pharmaceutical composition containing the anti-cancer agent, which may be administered. In this respect, the pharmaceutically acceptable carrier is not particularly limited, and carriers capable of improving the efficiency of permeation of the anti-cancer agent of the present invention into target cancer tissues or cancer cells, or the like may be preferably used.

The carrier may include cationic liposomes for preventing the double strand siRNA from degradation in a living body, and for increasing permeability into the cells (for example, see Yano J. et al., Clin Cancer Res., 10 (22), p. 7721-7726 (2004)). In addition, the double strand siRNA that constitutes the anti-cancer agent of the present invention may be chemically modified in order to prevent the degradation in a living body (for example, see Rossi J. J., Nature, 432 (7014), p. 155-156 (2004); Soutschek J. et al., Nature, 432 (7014), p. 173-178 (2004) and the like).

The formulation of the pharmaceutical composition of the present invention is not particularly limited, and examples the formulation include injections, creams, ointments, suspensions, and the like.

### Method for Inducing Apoptosis of Cancer Cells

The present invention also relates to a method for inducing apoptosis of cancer cells in which the anti-cancer agent of the present invention is used. The method for inducing apoptosis of cancer cells is performed by bringing the anti-cancer agent of the present invention into contact with isolated cancer cells or a cell population including cancer cells.

In the step of bringing the anti-cancer agent of the present invention into contact according to the method for inducing apoptosis of cancer cells of the present invention, a cell population containing at least cancer cells is brought into contact with a solution containing the anti-cancer agent of the present invention at a concentration of 50 to 80%, and the mixture is left to stand at 36 to 37°C for 4 to 72 hrs. By carrying out such an operation, the double strand siRNA constituting the anti-cancer agent of the present invention is incorporated into the cells, and thus the expression of RBM8A that is the protein set out in SEQ ID NO: 1, or Magoh that is the protein set out in SEQ ID NO: 3 is suppressed, whereby proliferation of cancer cells can be suppressed, and concurrently apoptosis of the cancer cells can be induced.

### Method for Screening for Anti-cancer Agent

The present invention also relates to a method for screening for an anti-cancer agent. The method for screening for an anti-cancer agent of the present invention includes: a step of allowing isolated cancer cells and candidate substances to be in contact (contacting step); a step of assaying expression and/or a function of a protein set out in SEQ ID NO: 1 or 3 or an isoform thereof in the cancer cells (measuring step); and a step of selecting a candidate substance that reduces the expression and/or the function of the protein set out in SEQ ID NO: 1 or 3 or an isoform thereof from the candidate substances (selecting step).

### (Contacting Step)

In the contacting step, the isolated cancer cells and candidate substances make contact. The cancer cells for use in the contacting step are not particularly limited, and for example, lung cancer cells, cervical cancer cells, pancreatic cancer cells and the like may be exemplified. In the process for allowing the cancer cells and the candidate substances to be in contact, cancer cells may be cultured in the presence of the candidate substances, or the candidate substances may be produced in the cancer cells.

For example, in the case in which cancer cells are cultured in the presence of the candidate substances, a method including: culturing the cancer cells in a 96-well microplate or the like; adding thereto the candidate substances; culturing the mixture at 36 to 37°C for 4 to 72 hrs; and then removing the candidate substance therefrom may be employed.

In the case in which the candidate substance is a double strand siRNA, the method may include introducing into a vector a DNA fragment that produces a double strand siRNA being the candidate substance under a predetermined condition; and inducing the production of the double strand siRNA in cancer cells into which the vector was introduced.

Typical candidate substance includes, for example, a double strand siRNA having a 3' overhang, in which at least one of RNA molecules that constitute the double strand siRNA is capable of complementarily binding to mRNA set out in SEQ ID NO: 2 or 4. In this instance, the candidate substance may be chemically or enzymatically synthesized in vitro, or may be synthesized in vivo. In particular, when enzymatically synthesized in vitro, a cDNA of RBM8A may be used as a source material for the synthesis by carrying out a restriction enzyme reaction or a ligation reaction.

### (Measuring Step)

In the measuring step, expression and/or a function of a protein set out in SEQ ID NO: 1 or 3 or an isoform thereof is assayed in cancer cells. The method for assaying the expression of the protein set out in SEQ ID NO: 1 or 3 or an isoform thereof is not particularly limited, and a western blotting method in which the expression level of a protein is measured, a Northern blotting method in which the expression level of an mRNA is measured, as well as antibody staining method and the like may be exemplified.

As a method for assaying a function of the protein set out in SEQ ID NO: 1 or 3 or an isoform thereof, a method in which RBM8A that is a protein set out in SEQ ID NO: 1, or Magoh that is a protein set out in SEQ ID NO: 3 is precipitated from an extraction liquid of cancer cells by, for example, an immunoprecipitation method, and the presence or absence of the protein that is coprecipitated with RBM8A or Magoh is checked by a western blotting method to detect the presence or absence of the interaction and complex formation of RBM8A or Magoh with other protein, or a method in which RBM8A or Magoh and a protein which has been known to interact therewith are stained by an antibody staining method to confirm as to whether or not both are colocalized, and the like may be exemplified.

Particularly, when a western blotting method or an antibody staining method is used, the expression level of the protein is preferably measured using an antibody for the protein set out in SEQ ID NO: 1 or 3 in the measuring step. In this instance, the antibody used may be either a monoclonal antibody or a polyclonal antibody, and a monoclonal antibody is preferably used.

### (Selecting step)

In the selecting step, a candidate substance that reduces the expression and/or the function of the protein set out in SEQ ID NO: 1 or 3 or an isoform thereof is selected from the candidate substances. More specifically, in the selecting step, depending on the procedure actually carried out in the measuring step, a procedure of selecting a candidate substance that decreases an expression level of a gene that expresses an mRNA set out in SEQ ID NO: 2 or 4, or selecting a candidate substance that reduces the interaction or complex formation of the protein set out in SEQ ID NO: 1 or 3 with the other protein may be carried out, and depending on the method carried out in the measuring step, a candidate substance may be selected by a conventionally well-known selecting method.

### EXAMPLES

Hereinafter, Examples of the present invention are described in detail with reference to the drawings. However, the present invention is not in any way limited by the Examples shown below.

In the following Examples, five types as described in the following were used as the double strand siRNA that inhibits RBM8A.

RBM8A-siRNA (IG-B): a double strand siRNA obtained by pairing an RNA strand that includes an RNA set out in SEQ ID NO: 5 having an overhang consisting of a thymidine ribonucleotide dimer at the 3' end, with an RNA strand that includes a complementary strand of the RNA set out in SEQ ID NO: 5 having an overhang consisting of a thymidine ribonucleotide dimer at the 3' end.

RBM8A-siRNA (IG-F): a double strand siRNA obtained by pairing an RNA strand that includes an RNA set out in SEQ ID NO: 6 having an overhang consisting of a thymidine ribonucleotide dimer at the 3' end, with an RNA strand that includes a complementary strand of the RNA set out in SEQ ID NO: 6 having an overhang consisting of a thymidine ribonucleotide dimer at the 3' end.

RBM8A-siRNA (1) : a double strand siRNA obtained by pairing an RNA strand that includes an RNA set out in SEQ ID NO: 7 having an overhang consisting of a thymidine ribonucleotide dimer at the 3' end, with an RNA strand that includes a complementary strand of the RNA set out in SEQ ID NO: 7 having an overhang consisting of a thymidine ribonucleotide dimer at the 3' end (manufactured by Invitrogen Corporation, product code "HSS115051").

RBM8A-siRNA (2): a double strand siRNA obtained by pairing an RNA strand that includes an RNA set out in SEQ ID NO: 8 having an overhang consisting of a thymidine ribonucleotide dimer at the 3' end, with an RNA strand that includes a complementary strand of the RNA set out in SEQ ID NO: 8 having an overhang consisting of a thymidine ribonucleotide dimer at the 3' end (manufactured by Invitrogen Corporation, product code "HSS115052").

RBM8A-siRNA (3): a double strand siRNA obtained by pairing an RNA strand that includes an RNA set out in SEQ ID NO: 9 having an overhang consisting of a thymidine ribonucleotide dimer at the 3' end, with an RNA strand that includes a complementary strand of the RNA set out in SEQ ID NO: 9 having an overhang consisting of a thymidine ribonucleotide dimer at the 3' end (manufactured by Invitrogen Corporation, product code "HSS115053").

Moreover, two types as described in the following were used as the double strand siRNA that inhibits Magoh.

Magoh-siRNA (1) : a double strand siRNA obtained by pairing an RNA strand that includes an RNA set out in SEQ ID NO: 10 having an overhang consisting of a thymidine ribonucleotide dimer at the 3' end, with an RNA strand that includes a complementary strand of the RNA set out in SEQ ID NO: 10 having an overhang consisting of a thymidine ribonucleotide dimer at the 3' end (manufactured by Invitrogen Corporation, product code "HSS142861").

Magoh-siRNA (2): a double strand siRNA obtained by pairing an RNA strand that includes an RNA set out in SEQ ID NO: 11 having an overhang consisting of a thymidine ribonucleotide dimer at the 3' end, with an RNA strand that includes a complementary strand of the RNA set out in SEQ ID NO: 11 having an overhang consisting of a thymidine ribonucleotide dimer at the 3' end (manufactured by Invitrogen Corporation, product code "HSS142862").

Also, as a control double strand siRNA, the following five types were used.
control-siRNA (L): "Stealth (registered trademark) RNAi Negative Control Low GC Duplex" (trade name, manufactured by Invitrogen Corporation)
control-siRNA (M): "Stealth (registered trademark) RNAi Negative Control Med GC Duplex" (trade name, manufactured by Invitrogen Corporation)
control-siRNA (H): "Stealth (registered trademark) RNAi Negative Control Hi GC Duplex" (trade name, manufactured by Invitrogen Corporation)
control-siRNA (SNC2): manufactured by Takara Bio Inc., product code "SNC2"
control-siRNA (SNC5): manufactured by Takara Bio Inc., product code "SNC5"

### Example 1: Identification of intracellular localization of RBM8A

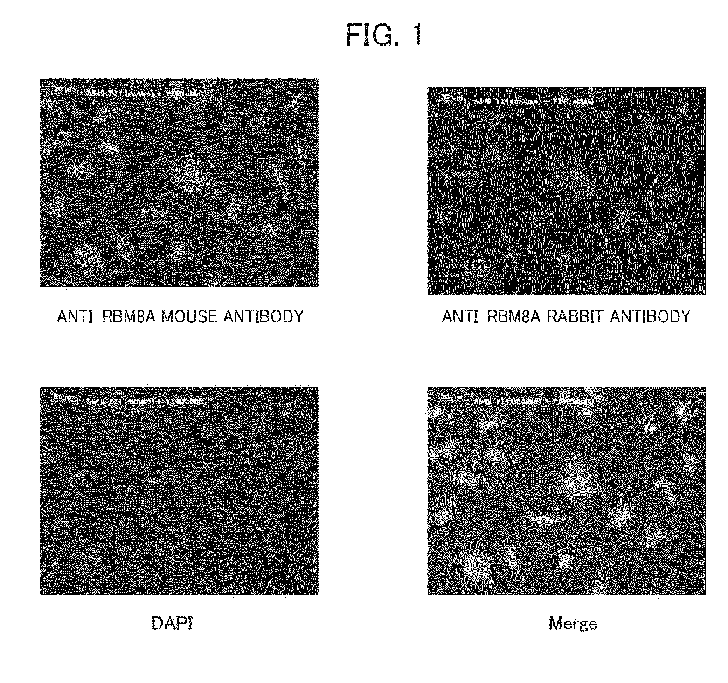

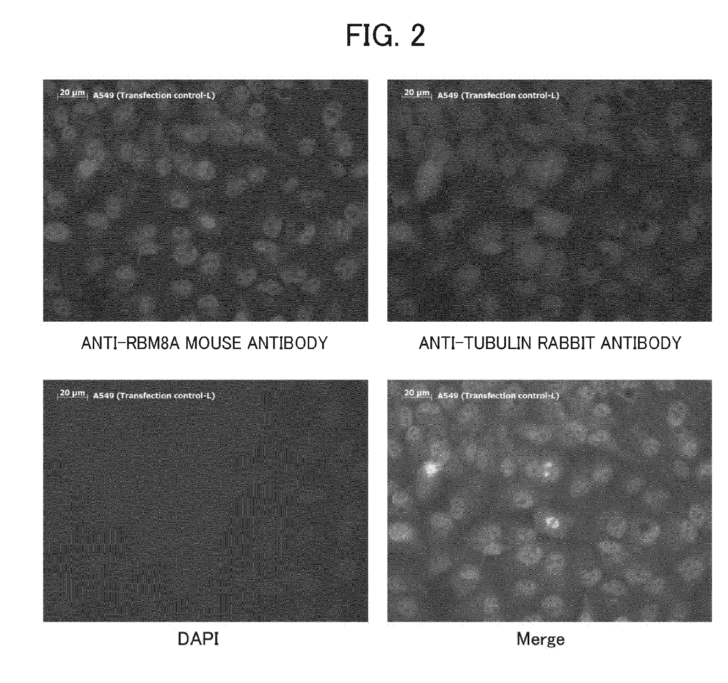

In identifying localization of RBM8A in cells, A549 cells which had been cultured in a 10% fetal bovine serum-supplemented Dulbecco's Modified Eagle's Medium at 37°C for 16 hours were used. A549 cells after the culture were washed with phosphate buffered physiological saline (PBS) three times, and thereafter fixed with a 4% paraformaldehyde solution, or methanol cooled to -20°C. The fixed A549 cells were treated with a 0.2% solution of Triton (registered trademark) X100, washed with PBS, and blocked with a 1% bovine serum albumin (BSA) solution. Thereafter, as a primary antibody, an anti-RBM8A mouse or rabbit antibody, an anti-α-tubulin rat antibody, an anti-γ-tubulin rabbit or mouse antibody, an anti-phosphorylated A urora B rabbit antibody, an anti-Magoh goat antibody, or an anti-FLAG peptide mouse antibody was used, and the blocked A549 cells were treated with a solution prepared by diluting the antibody. Subsequently, RBM8A, or RBM8A and various types of subcellular organelles were stained using an anti-rabbit IgG antibody and/or an anti-rat IgG antibody as a secondary antibody. After the stained culture cells were counter stained by adding 4',6-diamidino-2-phenylindole (DAPI), they were mounted using "Prolong Gold antifade Reagents" (trade name, mounting medium, manufactured by Invitrogen Corporation). The mounted sample "LSM710" or "Axiovert 200M" (both trade names, fluorescence microscope, manufactured by Carl Zeiss AG) was used to identify the localization of RBM8A and each subcellular organelle. Fluorescence microscopic views obtained by staining RBM8A alone with the antibody alone are shown in Fig. 1; and fluorescence microscopic views obtained by double staining RBM8A and γ-tubulin are shown in Fig. 2.

As shown in Fig. 1, the staining pattern of RBM8A overlapped with the staining pattern of DAPI, revealing that RBM8A is localized in the nucleus in intermitotic cells. Also, cells in the M phase exhibited a localization pattern similar to that of spindles, suggesting the interaction of RBM8A with spindle fibers.

Furthermore, as is seen from Fig. 2, RBM8A is colocalized with tubulin particularly in the M phase, suggesting that RBM8A is localized at the spindle.

### Example 2: Suppression of RBM8A Expression by Double Strand siRNA 1

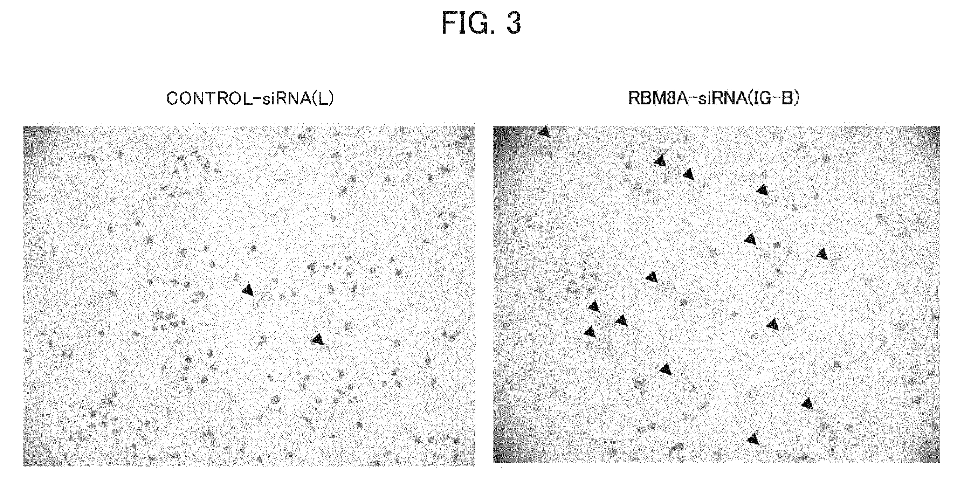

RBM8A-siRNA (IG-B) or control-siRNA (L) was transfected into A549 cells which had been cultured in a 10% fetal bovine serum-supplemented Dulbecco's Modified Eagle's Medium at 37°C for 16 hours, and a trypsin treatment was carried out to recover cells. The cells were swollen with a hypotonic solution, and thereafter fixed with a Carnoy fluid. After the cell suspension liquid was dropped on a slide glass and dried, staining with a Giemsa staining liquid was carried out. The proportion of cells forming a chromosome was measured under an optical microscope. The results are shown in Fig. 3.

From Fig. 3, the proportion of cells in which aggregated chromosomes were observed was significantly higher in the cells transfected with the RBM8A-siRNA (IG-B), as compared with the cells transfected with control-siRNA (L). Thus, it was indicated that the cells were enriched at the M phase due to suppression of the RBM8A expression.

### Example 3: Suppression of RBM8A Expression by Double Strand siRNA 2

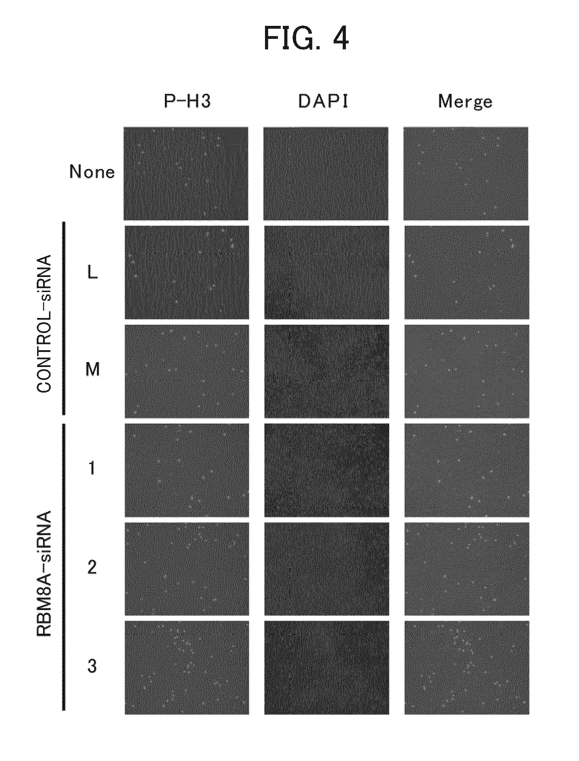

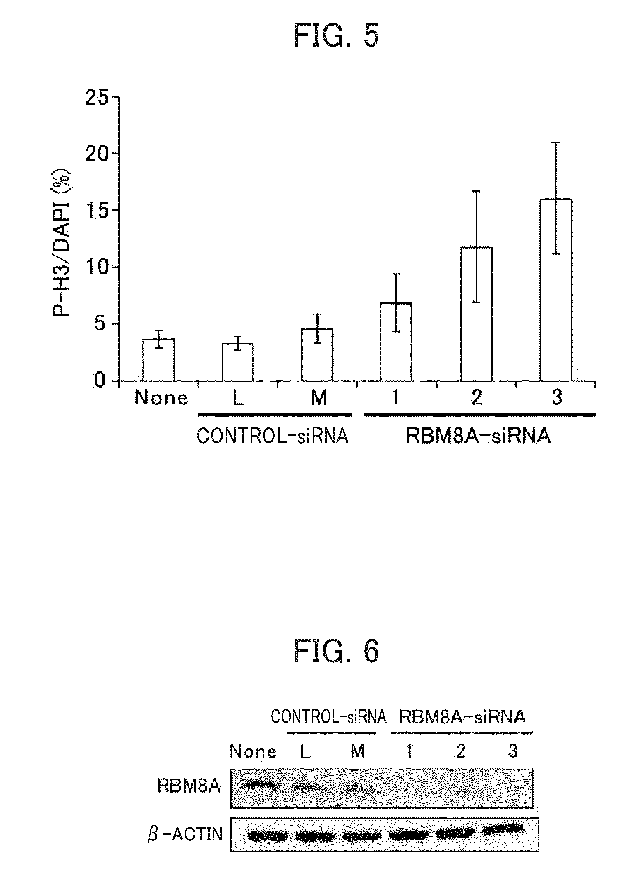

In a similar manner to Example 2, A549 cells were transfected with each of RBM8A-siRNA (1) to (3), and control-siRNA (L) and (M), and immunostaining was carried out in a similar manner to Example 1 using an anti-phosphorylated histone H3 antibody (anti-P-H3 antibody). The fluorescence microscopic views after staining are shown in Fig. 4. In addition, the proportion (%) of the cells stained with the anti-phosphorylated histone H3 antibody (cells in the M phase) with respect to the cells stained with DAPI is shown in Fig. 5. Furthermore, results of measurement of the RBM8A expression level in the cells transfected with each double strand siRNA are shown in Fig. 6.

As is seen from Fig. 4 to Fig. 6, in the cells transfected with each RBM8A-siRNA (1) to (3) to inhibit the RBM8A expression, the number of cells stained with the anti-phosphorylated histone H3 antibody was significantly greater as compared with cells transfected with control-siRNA (L) or (M). From these results, it was indicated that the cells were enriched at the M phase due to suppression of the RBM8A expression.

### Example 4: Suppression of RBM8A Expression by Double Strand siRNA 3

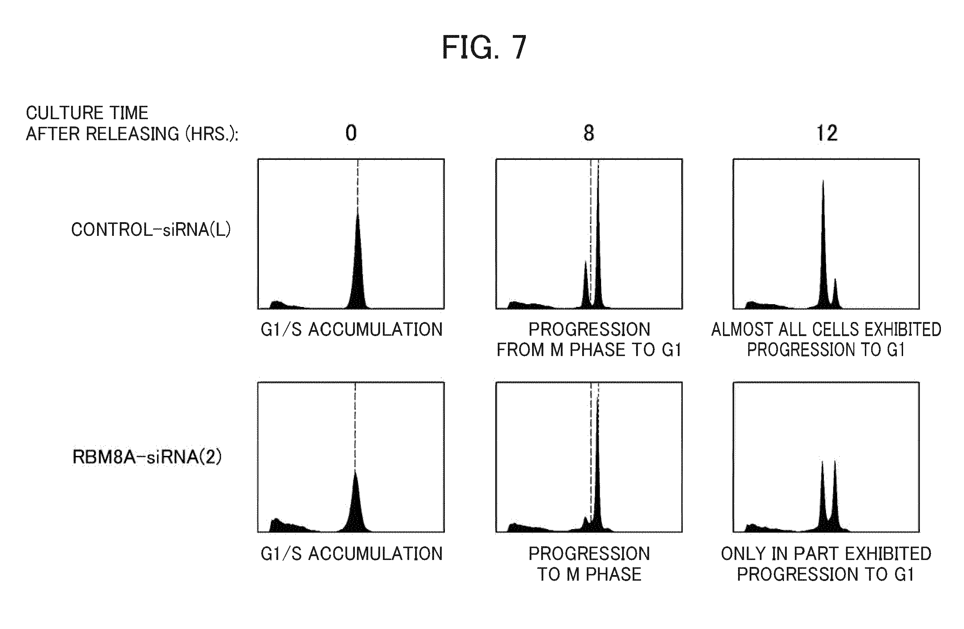

In a similar manner to Example 2, Hela cells were transfected with RBM8A-siRNA (2) or control-siRNA (L), and the cell cycle of the Hela cells was arrested at the G1/S phase by a double thymidine block method, followed by releasing. The proportion of cells that entered again into the G1 phase after passage of the G2/M phase in the cell cycle was determined with a flow cytometer "FACS Calibur" (trade name, manufactured by Becton, Dickinson and Co.). The results are shown in Fig. 7.

From Fig. 7, it is revealed that the proportion of the cells which exhibited progression of the G1 phase again after the M phase passage is significantly lower in the cells transfected with RBM8A-siRNA (2), as compared with cells transfected with control-siRNA (L). From this result, it is proven that the cell cycle tends to be arrested at the M phase in the cells transfected with RBM8A-siRNA (2).

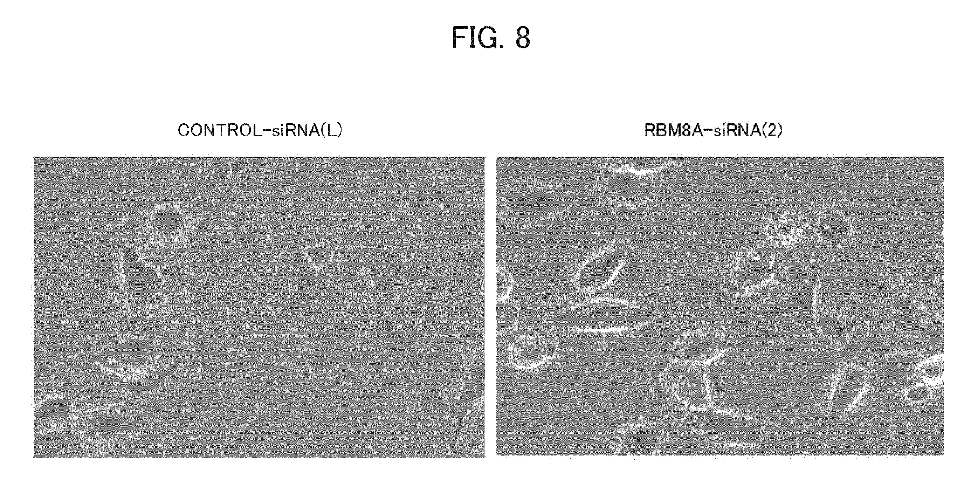

In addition, microscopic views of cells transfected with RBM8A-siRNA (2) or control-siRNA (L) when observed with a phase contrast microscope are shown in Fig. 8. As is seen from

Fig. 8, in the cells transfected with RBM8A-siRNA (2), the proportion of floating cells in which apoptosis had been induced was significantly greater as compared with the cells transfected with control-siRNA (L).

### Example 5: Suppression of RBM8A Expression by Double Strand siRNA 4

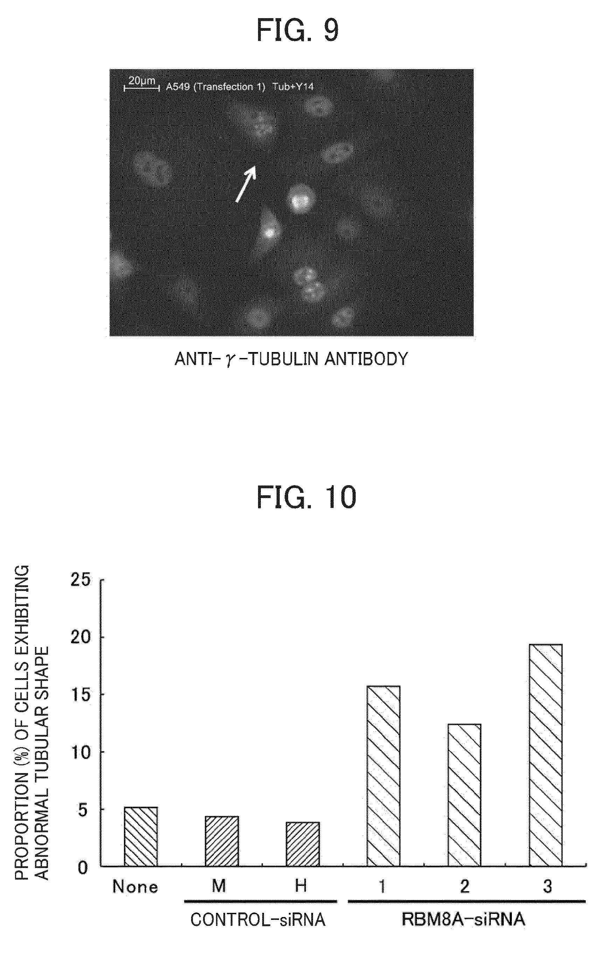

In a similar manner to Example 2, A549 cells were transfected with RBM8A-siRNA (2), and thereafter a trypsin treatment was carried out to recover the cells. Subsequently, γ-tubulin in the recovered cells was stained similarly to Example 1. Under a fluorescence microscope, localization of tubulin was confirmed. The antibody stained image with the anti-γ-tubulin antibody is shown in Fig. 9. In Fig. 9, white arrow indicates a cell showing an abnormal microtubular shape.

Similarly, γ-tubulin was stained with RBM8A-siRNA (1) to (3) and control-siRNA (M), (H), and the localization of tubulin was confirmed under a fluorescence microscope. The proportion (%) of cells that exhibit abnormal microtubular shape observed under a fluorescence microscope is shown in Fig. 10.

As is seen from Fig. 9 and Fig. 10, in the cells in which RBM8A expression had been suppressed by RBM8A-siRNA (1) to (3), many microtubules having an abnormal shape were observed. Example 6: Induction of Apoptosis by Double Strand siRNA

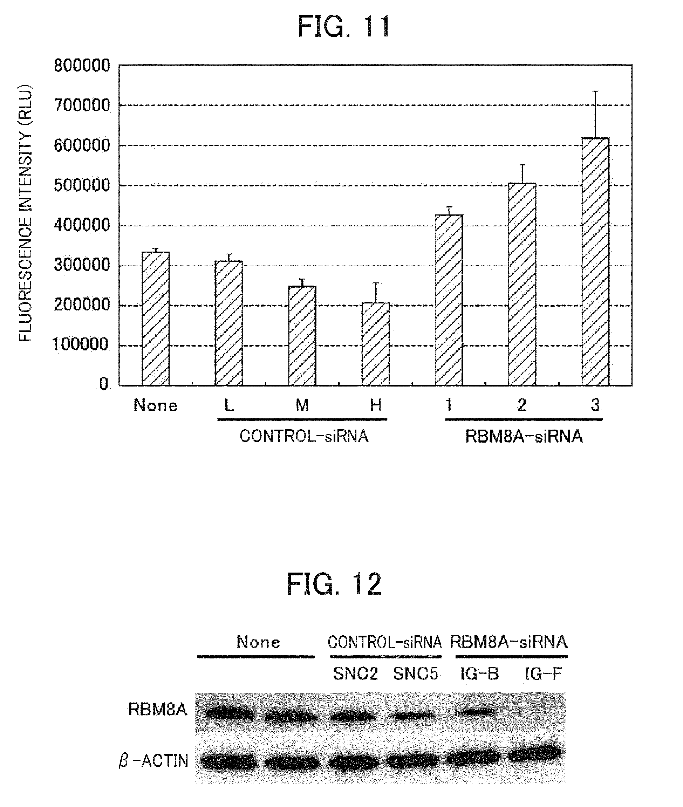

A549 cells were cultured in a 10% fetal bovine serum-supplemented Dulbecco's Modified Eagle's Medium at 37°C for 48 hours, and recovered by carrying out a trypsin treatment. Thereafter, the cells were adjusted to a state of single cell, and inoculated into a culture plate. Thereto each of RBM8A-siRNA (1) to (3), and control-siRNA (L), (M) and (H) was transfected, and the activity of caspase 3,7 was detected in a time dependent manner using "Apo-one Caspase-Glo 3/7 assay" (trade name, apoptosis detection kit, manufactured by Promega Corporation). The fluorescence intensity that indicates the activity level of caspase 3/7 in each cell is shown in Fig. 11.

As is seen from Fig. 11, in the cells transfected with each RBM8A-siRNA (1) to (3), the activity of caspase 3/7 increased as compared with cells transfected with control-siRNA (L), (M) or (H). From these results, apoptosis tends to be induced in the cells transfected with each RBM8A-siRNA, (1) to (3).

### Example 7: Suppression of RBM8A Expression by Double Strand siRNA 5

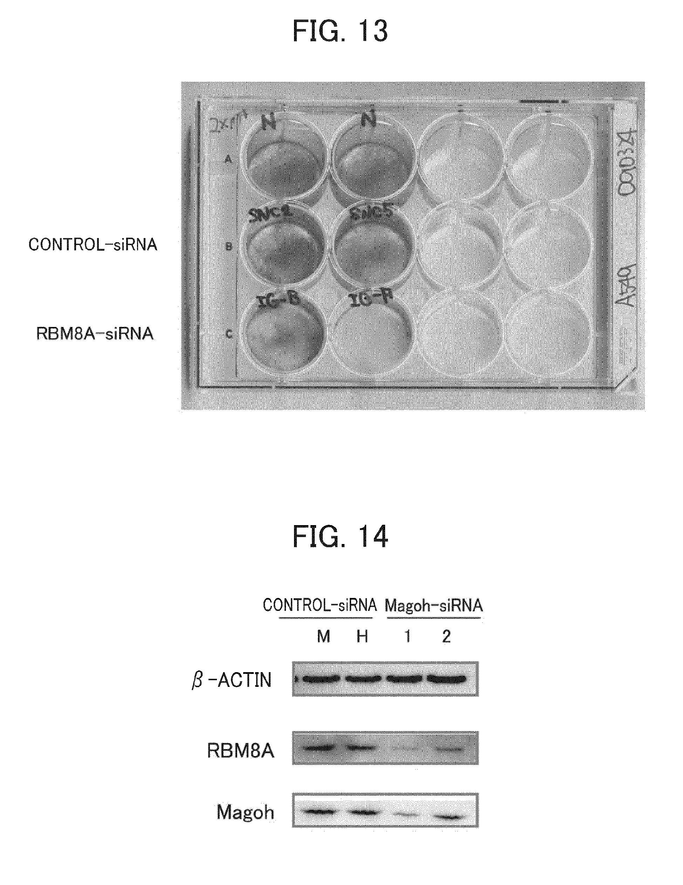

In a similar manner to Example 2, A549 cells were transfected with each of RBM8A-siRNA (IG-B) or (IG-F), or control-siRNA (SNC2) or (SNC5), and thereafter a trypsin treatment was carried out to recover the cells, which were then adjusted to a state of single cell and inoculated into a culture plate. The RBM8A expression level in each sample was measured, and the results are shown in Fig. 12. In addition, after the cells were cultured for two weeks, they were washed with PBS, and fixed in methanol and stained with a Giemsa staining liquid. The results are shown in Fig. 13.

From Fig. 12 and Fig. 13, in the cells transfected with RBM8A-siRNA (IG-B) or (IG-F) to suppress the RBM8A expression exhibited decrease of living cells. From this result, it was suggested that apoptosis was induced by suppression of the RBM8A expression.

### Example 8: Suppression of Magoh Expression by Double Strand siRNA 1

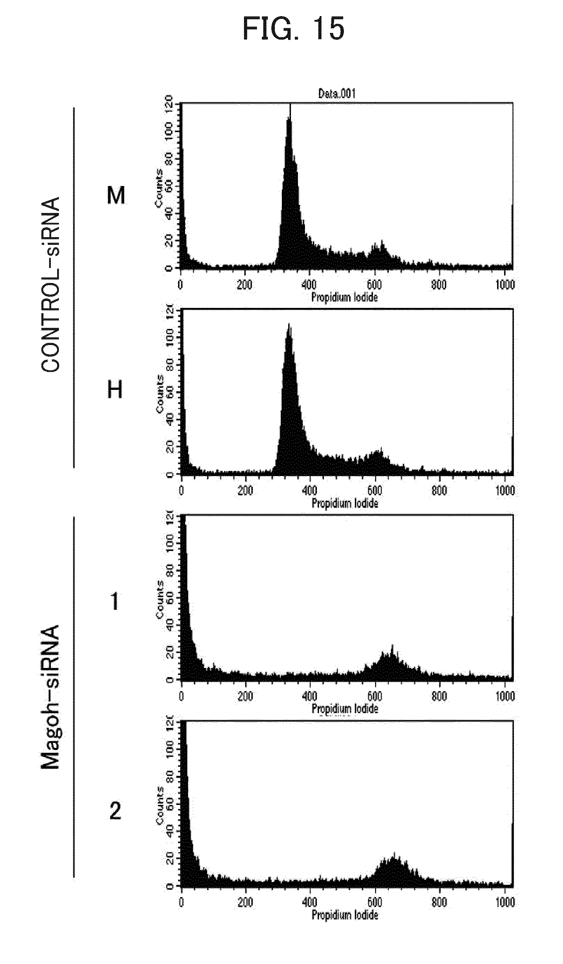

In a similar manner to Example 2, A549 cells were transfected with each of Magoh-siRNA (1) or (2), or control-siRNA (M) or (H), and thereafter a trypsin treatment was carried out to recover the cells. RBM8A and Magoh expression levels in each sample were measured, and the results are shown in Fig. 14. Furthermore, the cell cycle was analyzed using a flow cytometer "FACS Calibur" (trade name, manufactured by Becton Dickinson and Co.), and the results are shown in Fig. 15.

As is seen from Fig. 14, in the cells transfected with Magoh-siRNA (1) or (2), not only the Magoh expression but also the RBM8A expression was suppressed. Also, as is seen from Fig. 15, the cells transfected with Magoh-siRNA (1) or (2) exhibited predominantly low values accumulated on the graph, as compared with those of cells transfected with control-siRNA (M) or (H). From these results, it was revealed that suppression of the Magoh expression also suppressed the RBM8A expression, indicating induction of apoptosis.

### INDUSTRIAL APPLICABILITY

The anti-cancer agent of the present invention can specifically inhibit RBM8A set out in SEQ ID NO: 1, or Magoh set out in SEQ ID NO: 3 expression. In this regard, since inhibition of expression and/or a function of RBM8A or Magoh in cancer cells allows the cell cycles to be arrested at the M phase, thereby capable of inhibiting growth of the cells, proliferation of cancer cells can be suppressed, and concurrently apoptosis of the cancer cells can be induced, leading to cure of the cancer.

## Claims

1. An anti-cancer agent comprising a compound that specifically suppresses expression and/or a function of a protein set out in SEQ ID NO: 1 or an isoform thereof.

2. The anti-cancer agent according to claim 1, wherein the compound is a double strand siRNA that specifically suppresses the expression of the protein set out in SEQ ID NO: 1 or an isoform thereof, and
at least one of RNA molecules that constitute the double strand siRNA is capable of complementarily binding to an mRNA set out in SEQ ID NO: 2.

3. An anti-cancer agent comprising a compound that specifically suppresses expression and/or a function of a protein set out in SEQ ID NO: 3 or an isoform thereof.

4. The anti-cancer agent according to claim 3, wherein the compound is a double strand siRNA that specifically suppresses the expression of the protein set out in SEQ ID NO: 3 or an isoform thereof, and
at least one of RNA molecules that constitute the double strand siRNA is capable of complementarily binding to an mRNA set out in SEQ ID NO: 4.

5. The anti-cancer agent according to any one of claims 1 to 4, for use in a treatment of non-small cell lung cancer or breast cancer.

6. A pharmaceutical composition comprising as an active ingredient the anti-cancer agent according to any one of claims 1 to 5.

7. A method for inducing apoptosis of cancer cells, the method comprising using the anti-cancer agent according to any one of claims 1 to 5.

8. A method for screening for an anti-cancer agent, the method comprising: allowing isolated cancer cells and candidate substances to be in contact;
assaying expression and/or a function of a protein set out in SEQ ID NO: 1 or 3 or an isoform thereof in the cancer cells; and
selecting a candidate substance that reduces the expression and/or the function of the protein set out in SEQ ID NO: 1 or 3 or an isoform thereof from the candidate substances.

9. The method for screening for an anti-cancer agent according to claim 8, wherein: in the step of assaying expression and/or a function of a protein set out in SEQ ID NO: 1 or 3 or an isoform thereof, interaction or complex formation of the protein set out in SEQ ID NO: 1 or 3, and other protein that interacts with the protein set out in SEQ ID NO: 1 or 3 in a living body is detected; and
in the step of selecting a candidate substance that reduces the expression and/or the function of the protein set out in SEQ ID NO: 1 or 3 or an isoform thereof from the candidate substances, a candidate substance that reduces the interaction or complex formation of the protein set out in SEQ ID NO: 1 or 3 and the other protein is selected.

10. The method for screening for an anti-cancer agent according to claim 8, wherein in the step of assaying expression and/or a function of a protein set out in SEQ ID NO: 1 or 3 or an isoform thereof, an expression level of a gene that expresses an mRNA set out in SEQ ID NO: 2 or 4 is measured; and
in the step of selecting a candidate substance that reduces the expression and/or the function of the protein set out in SEQ ID NO: 1 or 3 or an isoform thereof from the candidate substances, a candidate substance that decreases an expression level of a gene that expresses an mRNA set out in SEQ ID NO: 2 or 4 is selected.

11. The method for screening for an anti-cancer agent according to claim 10, wherein the candidate substance is a double strand siRNA having a 3' overhang, and
at least one of RNA molecules that constitute the double strand siRNA is capable of complementarily binding to mRNA set out in SEQ ID NO: 2 or 4.

12. The method for screening for an anti-cancer agent according to any one of claims 8 to 11, wherein in the step of allowing isolated cancer cells and candidate substances to be in contact, the cancer cells are cultured in the presence of the candidate substance.

13. The method for screening for an anti-cancer agent according to any one of claims 8 to 12, wherein in the step of assaying expression and/or a function of a protein set out in SEQ ID NO: 1 or 3 or an isoform thereof, expression and/or a function of the protein is determined using an antibody to the protein set out in SEQ ID NO: 1 or 3.
